Europäisches Patentamt

European Patent Office

Office européen des brevets

(11) Publication number: **0 263 716**
A2

(12) **EUROPEAN PATENT APPLICATION**

(21) Application number: 87308957.7

(22) Date of filing: 09.10.87

(51) Int. Cl.⁴: **C 12 N 15/00**
C 12 N 1/20, C 12 P 19/32
//(C12N1/20,C12R1:19)

(30) Priority: 09.10.86 JP 240558/86
26.11.86 JP 281589/86

(43) Date of publication of application:
13.04.88 Bulletin 88/15

(84) Designated Contracting States: DE FR GB

(71) Applicant: **KYOWA HAKKO KOGYO KABUSHIKI KAISHA**
**6-1, Ohte-machi 1-chome**
**Chiyoda-ku Tokyo 100 (JP)**

(72) Inventor: **Fujio, Tatsuro**
**6-29-1, Sagamidai**
**Sagamihara-shi Kanagawa-ken (JP)**

**Nishi, Tatsunari**
**3-9-11, Naka-machi**
**Machida-shi Tokyo-to (JP)**

**Maruyama, Akihiko**
**3150, Noborito Tama-ku**
**Kawasaki-shi Kanagawa-ken (JP)**

**Ito, Seiga**
**218-14, Aza Hachiman-nishi Aihara**
**Sagamihara-shi Kanagawa-ken (JP)**

(74) Representative: **Hildyard, Edward Martin et al**
**Frank B. Dehn & Co. Imperial House 15-19 Kingsway**
**London WC2B 6UZ (GB)**

(54) A process for the preparation of 5'-guanylic acid and expression vectors for use therein.

(57) 5'-guanylic acid may be produced with higher yield by an enzymatic reaction using, as a source of enzyme, fermented product such as fermented liquor and microbial cells obtained by culturing a transformant of Escherichia coli which is capable of producing 5'-guanylic acid from 5'-xanthylic acid, adenosine-5'-triphosphate and at least one substrate selected from ammonia and glutamine and which is produced by transformation of a strain of E. coli carrying a temperature-sensitive cl repressor with a novel recombinant plasmid prepared by recombination of a DNA fragment carrying the $P_L$ promoter located upstream of a gene of 5'-guanylic acid synthetase with a vector DNA. It is possible to culture the transformed microorganism in a medium containing 5'-xanthylic acid, adenosine-5'-triphosphate and at least one member selected from ammonia and glutamine.

EP 0 263 716 A2

**Description**

A PROCESS FOR THE PREPARATION OF 5'-GUANYLIC ACID AND EXPRESSION VECTORS FOR USE THEREIN

The present invention relates to a process for the preparation of 5'-guanylic acid (hereinafter referred to as GMP) and to expression vectors prepared by means of genetic recombination of DNA for use therein. More particularly, the present invention relates to a process for the preparation of GMP by the enzymatic reaction, in which a transformant is cultured in a medium to obtain a fermented liquor, microbial cells or materials which are obtained by treating said fermented liquor or microbial cells, and then said fermented liquor, microbial cells or material is used as the source of enzyme for the reaction to convert 5'-xanthylic acid (hereinafter referred to as XMP) into GMP, said transformant being obtained by the use of DNA which is prepared by recombination of a DNA fragment carrying a gene of guanylic acid-synthetase (hereinafter designated as GMP synthetase) capable of synthesizing GMP from XMP, adenosine-5'-triphosphate (hereinafter designated as ATP) and at least one member selected from ammonia and glutamine with a fragment of vector DNA. GMP is very useful for the preparation of taste-enhancing agents and it is highly desirable to provide a cheaper process for producing GMP other than by conventional methods.

Examples of the known processes for the preparation of GMP include the decomposition of ribo-nucleic acid, the production of a precursor such as, for example, guanosine, from which GMP is obtained by chemical phophorylation, and the direct production of GMP by fermentation of microorganism.

The present inventors have studied the preparaation of GMP from XMP and have disclosed in Japanese Patent Application laid open to public inspection as 192597/85 that a transformed microorganism exhibits a high ability to produce GMP from XMP, said microorganism being prepared by the steps of cloning a gene of GMP synthetase which is an enzyme capable of producing GMP from XMP (in some cases hereinafter referred to to as guaA) by using a plasmid vector pBR322, thereafter forming a recombinant plasmid by recombining the plasmid vector with a DNA fragment carrying a promoter of tryptophan operon of Escherichia coli (hereinafter referred to as trp promoter or Ptrp) located upstream of guaA, and transforming a microorganism by the use of said recombinant DNA.

As a result of our further study, it has now been found that a microorganism having a higher ability to convert XMP into GMP may be obtained by transformation of Escherichia coli carrying a gene of a temperature-sensitive cl repressor (cl$_{857}$) by the use of a plasmid vector, into which is inserted a DNA fragment carrying guaA downstream of the P$_L$promoter.

The present invention provides a process for the preparation of GMP, by which said GMP is produced from XMP, ATP and at least one member selected from ammonia and glutamine by enzymatic reaction, which process comprises

preparing a recombinant DNA by inserting a fragment of DNA carrying a gene of GMP synthetase downstream of a P$_L$ promoter into a vector DNA,

transforming Escherichia coli carrying a temperature-sensitive cl repressor by the use of said recombinant DNA,

culturing the transformant in a medium,

either carrying out said enzymatic reaction by using as the source of enzyme, one member selected from fermented liquor, microbial cells and materials which are obtained by treating said fermented liquor or said microbial cells,

or culturing said transformant in a medium containing XMP, ATP and at least one member selected from ammonia and glutamine,

and, if desired, isolating GMP from the resultant reaction solution or fermented liquor.

Further aspects of the invention provide an Escherichia coli expression vector carrying the genetic information for an enzyme capable of catalysing the conversion of 5'-xanthylic acid into 5'-guanylic acid and, upstream thereof, the P$_L$-promoter and Escherichia coli strains transformed with an expression according to the invention.

Examples of DNA fragments carrying a gene of GMP synthetase, which may be used for the purpose of the present invention include those derived from prokaryotes, bacteriophages or plasmids, although it is preferred to use DNA fragments carrying guaA originating from Escherichia coli, and examples of plasmids include those carrying such a DNA fragment. Thus, it is preferred to use pLC34-10 or pLC32-35, as the source of guaA which is a hybrid plasmid formed by recombining a DNA fragment carrying both a gene of inosinic acid-dehydrogenase (hereinafter referred to as guaB) and guaA (both originating from the chromosome of Escherichia coli) with colicin E, (hereinafter referred to as ColE,) DNA [all disclosed in Methods in Enzymology, 68, 296-408 (1979)].

Hereinafter, Escherichia coli JA200 which is available from E. coli Genetic Stock Center, Yale University, [cf. L. Clarke & J. Carbon, Cell 9, 91-99 (1976)] was used to separate and purify the desired plasmid DNA.

Also, pXAR66 plasmid is preferred, which is produced by the steps of removing a major portion of guaB gene from pLC34-10 and inserting a tryptophan promoter into the upstream of the DNA fragment carrying the remainder of the guaA gene (disclosed in Japanese Patent Application laid open to public inspection as No. 192597/85).

On the chromosome of E. coli, guaA is located downstream of guaB, and both guaA and guaB form an operon in conjunction with the region of the promoter-operator located upstream of guaB [Molec. Gen. Genet. 147, 203-208 (1976)]. Where GMP is accumulated in the microbial cells, the expression of guaA and guaB genes may be inhibited. Also, as is known, there is a secondary promotor interposed between guaA and guaB, of which transcription activity is lower than the corresponding activity of the promotor located in the upstream region [J. Bact. 131, 685-688 (1977)].

Any and all plasmids may be used to prepare the recombinant plasmid according to the present invention provided the expression of the GMP synthetase gene in Escherichia coli is possible. However, it is preferred to use those capable of imparting, for example, resistance to antibiotics such as, for example, pPLDI, pP$_L$T4, pP$_L$T20, pPAI and pPLA66 plasmids. which carry p$_L$-promoter and which are derived from pBR322 [Gene. 2, 95 (1977)] and pBR325 [Gene. 4, 121 (1978)].

The preparation of recombinant plasmid from a DNA fragment carrying the gene and the vector DNA may be effected in vitro in conventional manner, for example, by the steps of physical or enzymatic cleavage of a DNA carrying a desired gene and vector DNA, followed by recombination using a ligase. The desired recombinant may be separated from the solution of the ligase reaction by the steps of direct transformation of Escherichia coli in a solution of the DNA mixture, and selecting a microorganism imparted with the characteristics originating from the genetic information of the desired genes.

Where a vector capable of making the host strain resistant to antibiotics such as, for example, pBR322 is used, the desired recombinant may be obtained by the steps of transformation, selection of the desired resistant strain and separation of the strain to which the transformed characteristics originating from the genetic information of the desired genes are imparted.

With regard to the host microorganisms which may be used for the purpose of the present invention, any and all strains of Escherichia coli may be used sofaras they carry a mutant gene having a temperature-sensitive cl repressor of λ -phage, for example, cl857. Examples of preferred host strains include Escherichia coli MP 347 (FERM BP-408) and E. coli Nr 69.

Hereinafter, transformation of the host strain using recombinant DNA is carried out in conventional manner, as disclosed in Cohen et al.: Proc. Natl. Acad. Sci. U.S.A. 69, 2110 (1972).

Where ampicillin-sensitive E. coli MP347 is used as a host strain, a recombinant carrying guaA which is capable of being manifested in the host cell may be obtained by the steps of transformation, selection of a ampicillin-resistant strains and further selection of a strain which is imparted with a higher activity of GMP synthetase.

By culturing the resultant transformant in conventional manner which is used for culturing strains of E. coli, it is possible to obtain a fermented liquor, microbial cells or materials which may be obtained by treating said fermented liquor or microbial cells. They exhibit a strong ability to produce GMP from XMP, ATP and at least one member selected from ammonia and glutamine.

Culturing may be effected in a medium containing sources of carbon, nitrogen, inorganic substances, amino acids, vitamins and the like under aerobic conditions at adjusted temperature, pH and the like.

Examples of carbon source include glucose, fructose, sucrose, maltose, mannitol, sorbitol and other carbohydrates : sugar alcohols, glycerol, starch hydrolyzate solution, molasses and the like, although it is possible to use organic acid such as, for example, pyruvic acid, lactic acid, citric acid, and amino acids such as, for example, glutamic acid, methionine and the like.

Examples of nitrogen source include ammonia, ammonium chloride, ammonium sulfate, ammonium carbonate, ammonium acetate and other organic and inorganic salts of ammonium: glutamic acid, glutamine, methionine and other amino acids; pepton, NZ-amine, corn steep liquor, meat extract, yeast extract, casein hydrolyzate, fish meal and its digested products, chrysalis hydrolyzate and other nitrogen-containing organic substances.

Examples of inorganic substances include potassium dihydrogen phosphate, sodium hydrogen phosphate, magnesium sulfate, sodium chloride, potassium chloride, iron chloride, copper sulfate, manganese chloride, ammonium molybdate, zinc sulfate and the like, which may, if desired be added to the medium. If vitamins, amino acids and various other substances required for the growth of the microorganism are contained in the above-mentioned components, it is not necessary to add them to the medium additionally.

Culturing may be effected under aerobic conditions with shaking or with aeration and agitation at a temperature of from 20 to 50°C (preferably 30-45°C). In order to obtain a higher GMP synthetase activity, it is advantageous to carry out culturing at an adjusted temperature of from 37 to 45 °C before or after the beginning of culturing which may usually be effected for 1 to 24 hours.

The resultant fermented product may be used, with or without after-treatment for placing in contact with XMP, ATP and at least one member selected from ammonia and glutamine.

The materials obtained by treating said fermented products are exemplified by concentrated or dried fermented broth, filtrate obtained by centrifugation of the product treated with acetone, surfactants, organic solvents, bacteriolytic enzymes and the like; immobilized cells, and enzyme extracted from the cells.

The enzymatic reation may be carried out in an aqueous solution by placing the starting materials into contact with the fermented product which may be used with or without after-treatment. For this purpose, it is preferred to culture the microorganism in a medium containing XMP, ATP and at least one member selected from ammonia and glutamine to accumulate GMP in the fermented liquor. In this case, if desired, it is possible to add a surfactant and/or an organic solvent in the medium.

Alternatively, after completion of culturing, XMP, ATP and at least one member selected from ammonia and glutamine may be added to the fermented liquor, microbial cells or materials obtained by treating the fermented product. The reaction may be effected at a temperature of from 20 to 50°C for a period of from 1 to 48 hours to accumulate GMP in the reaction solution. In such a case, it is possible, if desired, to add a surfactant and/or an organic solvent to the reaction solution.

The reaction may usually be effected at a pH of 6-10.

The medium and the reaction solution may contain, for example, XMP (1-100), ATP (1-100), $MgSO_4 \bullet 7H_2O$ (1-50), $(NH_4)_2 SO_4 \bullet 7H_2O$ (1-25) and glutamine (1-25) [unit: g/l].

Preferred examples of XMP which may be used for the purpose of the present invention include samples having a high purity, and products obtained by fermentation of XMP which may be used without aftertreatment or with the following concentration or partial purification. They may be used sofaras they contain XMP and do not give any adverse effect upon the accumulation of GMP.

Preferred examples of ATP which may be used for the purpose of the present invention include samples having high purity, solutions containing ATP which may be obtained by placing adenine into contact with microbial cells in the presence of a substance capable of supplying energy (disclosed in Japanese Patent Application laid open to public inspection as No. 51799/84), ATP-containing solution, from which microbial cells are removed, and its concentrate.

With regard to preferred surfactants, for example, polyoxyethylene stearylamine [for example, Naimin S-215, commercially available from Nihon Yushi K.K., Japan), cetyltrimethylammonium bromide and other cationic surfactants; sodium oleylamide sulfonic acid and other anionic surfactants; polyoxyethylenesorbitan monostearate [for example, Nonion ST221, commercial product of Nihon Ushi K.K., Japan], lauryl betaine [for example, Anon BF, commercial product of Nihon Yushi K.K., Japan) and other amphoteric surfactants may be used. Usually, these surfactants may used at a concentration of 0.1-50 g/l (preferably 1-20 g/l).

With regard to organic solvents, it is possible to use, for example, toluene, xylene, aliphatic alcohols, benzene and ethyl acetate at a concentration of 0.1-50 ml/l (preferably 1-20 ml/l).

GMP accumulated in aqueous solution may be collected in conventional manner such as, for example, by the use of active carbon, ion exchange resins and the like.

According to the process of the present invention it is possible to convert 5'-xanthylic acid (XMP) into 5'-guanylic acid (GMP) with high efficiency.

The following non-limiting examples illustrate the present invention.

Brief description of the drawings:

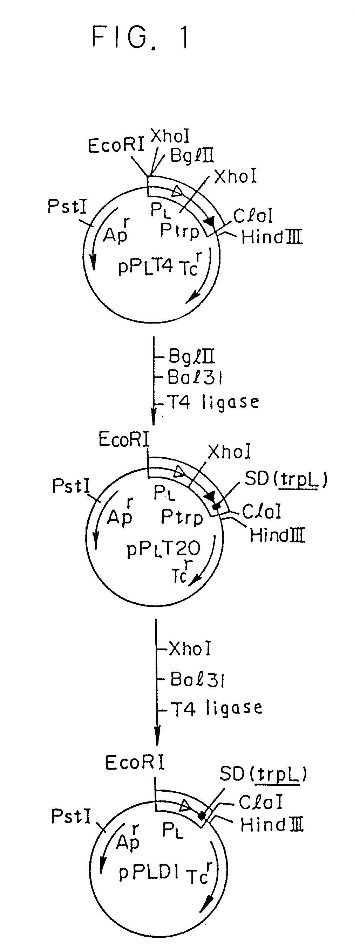

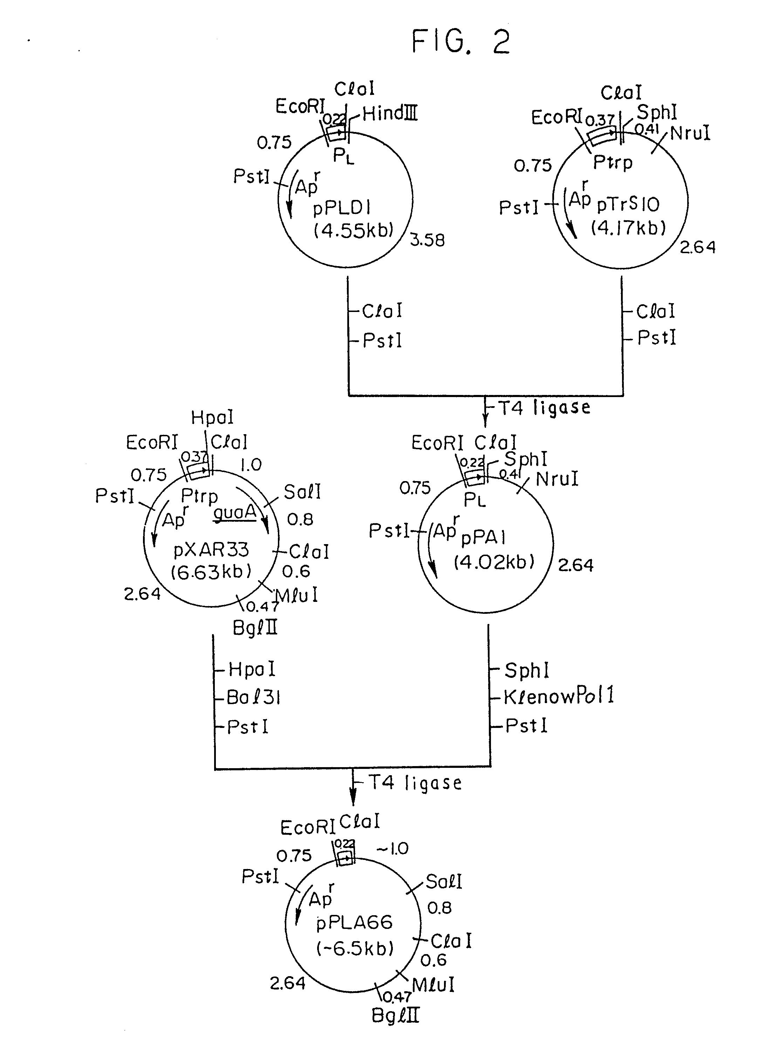

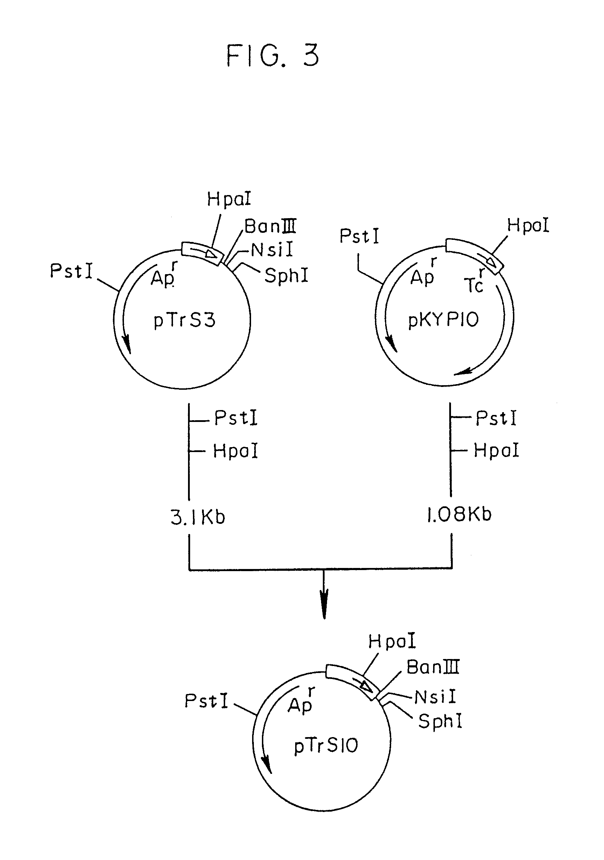

Fig. 1 shows schematically the preparation of plasmid pPLD 1. Fig. 2 shows schematically the preparation of plasmid pPLA 66. Fig. 3 shows schematically the preparation of plasmid pTrS 10.

Example 1

Preparation of a recombinant plasmid capable of manifesting GMP effectively:

(1) Preparation of $P_L$-ATG vector:

Escherichia coli 1$P_L$T4 (FERM-BP 406) containing a pP$_L$T4 plasmid vector, of which promoter portion had a $P_L$ promoter linked with tryptophan promoter (disclosed in Japanese Patent Application as laid open to public inspection as No. 126086/85), was inoculated to a medium (referred to as L medium) containing Bactotryptone (Difco., U.S.A.; 10 g/l). yeast extract (Difco.: 5 g/l), NaCl (5 g/l) and having a pH of 7.2 for culturing at 30 °C for 18 hours. From the resultant fermented liquor, plasmid pP$_L$T4 was separated and purified in a conventional manner as set forth.

In the following examples, L medium was used for culturing and maintaining the microorganism unless otherwise specified.

The resultant pP$_L$T4 plasmid DNA (about 5 μ g) was dissolved in a buffered solution (50 μ l) composed of tris-HCl (10 mM; pH 7.5), NaCl (100 mM), $MgCl_2$ (7mM) and 2-mercaptoethanol (6 mM). [This buffer solution is designated as Y-100 buffer solution. Buffer solutions of a similar composition except 50 mM and 0 mM of NaCl are designated as Y-50 and Y-0 buffer solutions respectively.]

To this solution was added a restriction enzyme (20 units of Bgl II; all restriction enzymes used herein are commercial products of Takara Shuzo K.K., Japan) to carry out the digestion at a temperature of 37 °C for 2 hours. To the resultant reaction solution (30 μ l) were added Bal 31 buffer solution (20 μ l; concentration X5) composed of tris-HCl (100 mM; pH 8.1), $MgCl_2$ (60 mM), $CaCl_2$ (60 mM) and NaCl (3 M), 48 μ l of of distilled water and one unit of Bal 31 (commercial product of Bethesda Research Laboratories, U.S.A.) for digestion at a temperature of 30°C for 10 seconds. A mixture of phenol and chloroform (1:1 v/v., 100 μ l) was added to the reaction solution with stirring to discontinue the reaction. After centrifugation, the upper layer was collected, to which was then added ice-cooled ethanol (amount X2). The mixture was allowed to stand at -80 °C for 30 minutes. After centrifugation of the mixture, the supernatant was removed. The precipitates were dissolved in 40 μ l of T4DNA ligase buffered solution composed of tris-HCl (20 mM;pH 7.6), $MgCl_2$ (10 mM), dithiothreitol (10 mM) and ATP (0.5 mM), followed by addition of T4 ligase (2 units). The mixture was treated at 4 °C for 18 hours to obtain a recombinant plasmid DNA which was then used to transform Escherichia coli MP347 by the method of Cohen et al, as set forth. The resultant transformed strain was resistant to ampicillin (50 μ g/ml).

Plasmid was recovered from this strain and was digested by means of restriction enzymes such as Xho I and Bgl II to analyze its structure. As a result, it was noted that the Xho I and Bgl II sites located upstream of the $P_L$

promoter were lost. This plasmid was designated as pP$_L$T20.

Further, pP$_L$T20 DNA (about 5 μ g) was dissolved in 50 μ l of Y-100 buffered solution, followed by adding Xho I restriction enzyme (20 units) for digestion at a temperature of 37 °C for 2 hours. After addition of 20 μ l of Bal 31 buffered solution (concentration X5), 48 ml of distilled water and one unit of Bal 31 to the solution (30μ l) of Xho I digestion, the mixture was subjected to digestion reaction carried out at a temperature of 30 °C for 3 minutes. The reaction solution was extracted with a mixture of phenol and chloroform (1:1 v/v) and precipitated by addition of ethanol. The resultant precipitates were added to 20μ l of T4DNA ligase-buffered solution to carry out the recirularisation reaction using 2 units of T4DNA ligase at a temperature of 4°C for 18 hours.

Escherichia coli MP347 was transformed to obtain an ampicillin-resistant strain by using the resultant recominant plasmid DNA. Among them, a strain resistant to 20 μ g/ml of tetracycline was selected and plasmid vector was collected from the selected strain. The base sequence of a fragment of Eco RI- Hind III including the P$_L$ promoter was determined by the method of Maxam-Gilbert [Proc.Natl. Acad. Sci., 74, 560 (1977)] to observe that the dimension of this fragment was 220 base pairs, in which only the portion of tryptophan promoter was digested and P$_L$promoter was retained. This plasmid which was designated as pPLD1 is shown in Fig. 1.

About 3 μ g of pTrS10 DNA carrying an initiation codon ATG was prepared by the method of Reference 1 described hereinafter, and was then dissolved in 50 μ l of Y-0 buffered solution (hereinbefore defined). To this solution was added 15 units of Cla I to carry out the digestion at a temperature of 37°C for 2 hours. To the reaction solution were added 0.5 μ l of NaCl (5 M) and 15 units of Pst I to carry out the digestion at a temperature of 37°C for 2 hours.

Low gelling temperature agarose gel electrophoresis (hereinafter referred to as the LGT method) described e.g. in Analytical Biochemistry, 98, 305 (1979) was used to purify DNA fragments hereinafter. In this manner, a DNA fragment of 3. 05 Kb including ATG portion was purified.

Separately, a similar procedure was used to digest about 3 μ g of pPLD1 DNA by the use of Cla I and Pst I, and then a fragment of DNA of 0.97 Kb including the P$_L$promoter was purified. About 0.5 μ g of the fragment of DNA derived from pTrS10 and about 0.2μ g of the fragment of DNA derived from pPLD1 were recombined in 20μ l of T4DNA ligase-buffered solution by the use of 2 units of T4 ligase. The reaction was effected at 4°C for 18 hours to obtain a recombinant plasmid which was then used to obtain an ampicillin-resistant strain by transformation of Escherichia coli MP347.

Plasmid collected from the resultant strain was purified and digested by the use of restriction enzymes such as, for example, Sph I, Cla I and Pst I to analyze its structure. A P$_L$-ATG vector of 4. 02 Kb including ATG portion in the downstream of P$_L$ promoter was noted, which was designated as pPA 1 and is shown in Fig. 2.

(2) Insertion of P$_L$-ATG sequence upstream of guaA gene:

About 5μ g of pPA 1 DNA was dissolved in 50 μl of Y-50 buffered solution. To this solution was added 20 units of Sph I to carry out the digestion at 37 °C for 2 hours. After extraction with a mixture of phenol and chloroform (1:1 v/v) and precipitation with ethanol, a fragment of DNA was dissolved in 50 μ l in total of DNA polymerase I buffered solution in 50 μ l in total of tris-HCl (pH 7.6: 50 mM), MgCl$_2$ (7 mM), 2-mercaptoethanol (6 mM), dATP (0.25 mM), dCTP (0.25 mM), dGTP (0.25 mM), and dTTP (0.25 mM). To the solution was added 8 units of E. coli DNA polymerase I • Klenow fragment (commercial product of Bethesda Research Laboratories, U.S.A) to carry out the reaction at 15°C for 2 hours so as to make 3'-protruding ends smooth. Extraction with chloroform was performed to discontinue the reaction.

After extraction with chloroform and precipitation with ethanol, the DNA fragment was dissolved in 50 μ l of Y-50 buffered solution. To the solution was added 20 units of Pst I to carry out the digestion at a temperature of 37 °C for 2 hours. After heat-treatment at 65 °C for 10 minutes, the solution was subjected to the LGT method to purify a fragment of DNA of 0.97 Kb including P$_L$ promoter portion and ATG portion.

Separately, about 5μ g of DNA of plasmid pXAR 33 carrying a tryptophan promoter and a gene of GMP synthetase (guaA) derived from Escherichia coli (disclosed in Japanese Patent Application laid open to public inspection as No. 192597/85) was dissolved in 50 μ l of Y-100 buffered solution, to which was then added 20 units of Hpa I to carry out the digestion at a temperature of 37°C for 2 hours.

To 30μ l of the reaction solution of Hpa I digestion reaction were added 20μ l of Bal 31 buffered solution (concentration X 5), 46μ l of distilled water and 2 units of Bal 31 to carry out the digestion at a temperature of 30 °C for 5 minutes.

After extraction with a mixture of phenol and chloroform and precipitation with ethanol, the DNA fragment was dissolved in 30 μ l of Y-50 buffered solution. To this solution was added 15 units of Pst I to carry out the digestion at a temperature of 37 °C for 2 hours. After heat-treatment at a tempeature of 65 °C for 10 minutes, the solution was subjected to the LGT method to purify a fragment of DNA of about 5.5 Kb, of which guaA was digested on the side of the N-terminal.

The fragment of DNA originating from pPA 1 (about 0.2 μ g) and the fragment of DNA originating from pXAR 33 (about 0.5 μ g) were recombined by treating with T4DNA ligase (20μ l) at a temperature of 4°C for 18 hours to obtain a recombinant plasmid DNA which was then used to transform Escherichia coli MP347.

There were obtained ampicillin-resistant strains which were then cultured at a temperature of 30 °C for 4 hours and further at a temperature of 40°C for 3 hours using L medium. Their GMP synthetase activities were investigated in a similar manner to that described hereinafter to select a strain exhibiting a higher activity than the activity of E. coli K294 carrying pXAR 33.

From a strain E. coli A-66 exhibiting the highest activity, a plasmid designated pPLA 66 was collected. Its structure was analyzed to observe that the P$_L$-ATG sequence was inserted upstream of the guaA gene.

In order to determine the GMP synthetase activity, a known method disclosed in J. Biol., 226, 351-363 (1957) was modified as follows:

In the cases of strains E. coli K294 and K294/pXAR used for determination, on each occasion, the strain was cultured at a temperature of 30 °C overnight by using L medium. 1 % of the seed culture was transformed to M9 medium for culturing at a temperature of 30 °C for 18 hours with shaking. M9 medium having the following composition:

$NH_4Cl$ 1 ; $Na_2HPO_4$ 6 ; $KH_2PO_4$ 3 ; NaCl 5; $MgSO_4 \cdot 7H_2O$ 0.25 ; glucose 3 ; vitamin B$_1$ 0. 004 ; casamino acid 2 (unit g/l).

Separately, strains MP 347 and MP347/pPLA 66 were cultured respectively at a temperature of 30 °C overnight by using L medium. On each occasion, 1 % of the seed culture was transferred to L medium for culturing at a temperature of 30° C for 4 hours and further at 40° C for 3 hours. The resultant fermented liquors were diluted with distilled water. After addition of toluene to the material to a final concentration of 20 ml/l, the solution was shaken at a temperature of 37° C for 20 minutes. Then the solution was diluted with water (X 1 ~ 100) according to the GMP synthetase acitivty. The thus-treated culture liquor was added to a reaction solution having the following composition: tris-HCl (160 mM; pH 8. 6) , ATP (12 mM) , XMP (25mM) , $MgSO_4 \cdot 7H_2O$ (16 mM) and $(NH_4)_2SO_4$ (40 mM).

The reaction solution was shaken at a temperature of 42 °C to convert XMP into GMP.

The reaction solution was sampled periodically to determine the formation of GMP by mixing a sample with perchloric acid (3.5 %; amount X40), centrifuging the mixture to collect the supernatant, and measuring its absorbancy at 290 nm.

Table 1 indicates GMP synthetase activities of the strains used or prepared by the process of the present invention, in which 1 unit denotes an activity capable of producing 1 μ mol of GMP per minutes.

### TABLE 1

| Host strain | Plasmid | Deposition No. (FERM-BP) | Activity* (unit/g of wet cell) |
|---|---|---|---|
| MP 347 | | 408 | 0.79 |
| K 294 | | 526 | 0.88 ** |
| K 294 | pXAR 33 | 500 | 73.5 ** |
| MP 347 | pPLA 66 | 1200 | 327 |

* GMP synthetase activity

** disclosed in Japanese Patent Application laid open to public inspection as No. 192597/85

The strain A-66 carrying plasmid pPLA 66 was deposited as Escherichia coli MP347/pPLA66 (FERM-BP 1200) with the Fermentation Research Institute, Agency of Industrial Science and Technology in Japan on 6th November 1986.

Example 2

The fermented liquor of a strain (hereinafter referred to as PLA66) carrying pPLA 66 produced by the method of Example 1 (containing 4. 8 mg per ml of wet cells) was concentrated to 20 times by centrifugation, to which was then added toluene to a final concentration of 20 ml/l. The mixture was shaken at a temperature of 37° C for 20 minutes. This cell suspension (0. 2 ml) was added to a reaction solution (30 ml) containing XMP• Na$_2 \cdot 7H_2O$ (20 mg/ml), ATP•Na$_2 \cdot 3H_2$) (20 mg/ml) and $(NH_4)_2SO_4$ (10mg/ml) and having an adjusted pH of 8. 6 (put in a 200 ml beaker). By using a magnetic stirrer, the reaction solution was stirred (900 r.p.m.) at a temperature of 42 °C for 3 hours, while its pH was kept at 8. 6 by adding caustic soda. By this reaction, 5′ -GMP• Na$_2 \cdot 7H_2O$ (15. 9 mg/ml) was produced in the reaction solution. Separately, a similar procedure was carried out by using the host strain MP347 instead of PLA66 to obtain not more than 4 mg/ml of the desired product.

Example 3

Strain PLA 66 was inoculated in L medium (30 ml) put in a 300 ml Erlenmeyer flask for culturing with shaking at a temperature of 30° C for 4 hours and further at 40 °C for 3 hours. To the fermented liquor were added toluene (20μ l/ml), XMP• Na$_2$• 7H $_2$O (20 mg/ml), ATP• Na$_2$•3H$_2$O ( 20 mg/ml) and $(NH_4)_2SO_4$ (10 mg/ml),

followed by further culturing at a temperature of 42 °C for 10 hours with shaking, while the pH was adjusted to 8. 6 with caustic soda. As a result, 17. 7 mg/ml GMP• Na₂• 7H₂O was accumulated in the fermented liquor. A similar procedure was carried out by the use of the host strain E. coli MP347 instead of strain PLA 66 to obtain not more than 1 mg/ml of the desired product.

## Reference 1

Preparation of ATG vector pTrS10:

With reference to the guideline shown in Fig. 3, ATG vector pTrS10 was prepared in the manner described hereinafter. The resultant vector had a portable Ptrp fragment of a dimension of 370 base pairs, and the interval between SD sequence and ATG intiation codon was 13 bases. Sph I site was located immediately after the ATG initiation codon.

About 3 $\mu$ g of pTrS3 (prepared by the method disclosed in Japanese Patent Application laid open to public inspection as No. 110600/83 was dissolved in Y-100 buffered solution (30$\mu$ l). To this solution were added 6 units each of restriction enzymes Pst I and Hpa I to carry out the digestion reaction at a temperature of 37 °C for 3 hours. The LGT method was applied to obtain about 2 $\mu$ g of a fragment of DNA of about 3. 1 Kb (fragment of Pst I-Hpa I) from the reaction solution.

Separately, 3 $\mu$ g of pKYP 10 (prepared by the method disclosed in Japanese Patent Application laid open to public inspection as No. 110600/83) was dissolved in Y- 100 buffered solution (30 $\mu$ l). To this solution were added 6 units each of restriction enzymes Pst I and Hpa I to carry out cleavage reaction at a temperature of 37 °C for 3 hours. The LGT method was applied to obtain about 0. 5 $\mu$ g of a fragment of DNA of about 1. 08 Kb (fragment of Pst I-Hpa I) from the reaction solution.

0. 1 $\mu$ g of the fragment of Pst I-Hpa I originating from pTrS3 and 0. 1 $\mu$ g of the fragment of Pst I-Hpa I of pKYP 10 origin were dissolved in 20 $\mu$ l of T4 ligase-buffered solution. To this solution was then added 2 units of T4DNA ligase to carry out the recombination at a temperature of 4°C for 18 hours.

The resultant mixture of recombinant plasmids was used for transformation of Escherichia coli HB 101 to obtain ampicillin resistant colonies . Plasmid DNA was prepared from a culture of the colonies and analyzed by the use of restriction enzymes Pst I, Hpa I, Ban III, Nsi I and Sph I to note that the desired plasmid was obtained.

## Claims

1. Escherichia coli expression vector containing the genetic information for an enzyme capable of catalyzing the conversion of 5′-xanthylic acid into 5′-guanylic acid and, upstream thereof, the P$_L$-promoter.

2. Escherichia coli expression vector as claimed in claim 1 wherein said vector contains the guaA gene under the transcriptional control of the P$_L$-promoter.

3. Escherichia coli expression vector as claimed in claim 2 designated pPLA66.

4. A strain of Escherichia coli transformed with an expression vector as claimed in any of claims 1 to 3, said strain carrying a temperature-sensitive cI repressor gene.

5. A strain of Escherichia coli as claimed in claim 4 which is resistant to ampicillin and tetracycline.

6. A strain of Escherichia coli as claimed in claim 5 designated MP347/pPLA66 (FERM-BP 1200).

7. A process for preparing 5′-guanylic acid wherein a strain of Escherichia coli as claimed in any of claims 4 to 6 is cultured in a fermentation medium to provide fermented liquor and microbial cells as fermented products, contacting an aqueous solution containing an effective amount of enzyme capable of catalyzing the conversion of 5′-xanthylic acid into 5′-guanylic acid and which is derived from at least one of said fermented products with 5′-xanthylic acid, adenosine-5′-triphosphate and at least one member selected from ammonia and glutamine, to produce 5′-guanylic acid.

8. A process as claimed in claim 7 wherein said 5′-guanylic acid is subsequently isolated.

9. A process as claimed in claim 7 or claim 8 wherein said culturing is effected at a temperature of from 20 to 50°C and at a pH of from 6 to 10 for 1 to 48 hours.

10. A process as claimed in any of claims 7 to 9 wherein said contact is effected by adding 5′-xanthylic acid, adenosine-5′-triphosphate and at least one member selected from ammonia and glutamine to the fermentation medium either before, during or after culturing.

11. A process as claimed in any of claims 7 to 10 wherein said culturing is carried out at an adjusted temperature of f rom 37 to 45°C, before or after the beginning of culturing, for 1 to 24 hours.

12. A process as claimed in any of claims 7 to 11 wherein said contact is effected in the presence of at least one member selected from a surfactant and an organic solvent.

13. A process for preparing an expression vector as claimed in any of claims 1 to 3 wherein the genetic information for an enzyme capable of catalysing the conversion of 5′-xanthylic acid into 5′-guanylic acid and the P$_L$-promoter are inserted into a vector such that said information can be expressed in a host Escherichia coli under the transcriptional control of said promoter.

14. A process for preparing a strain of Escherichia coli as claimed in any of claims 4 to 6 wherein a strain carrying a temperature-sensitive cl repressor gene is transformed with an expression vector as claimed in any of claims 1 to 3.

15. 5'-Guanylic acid prepared by a process as claimed in any of claims 7 to 12.

FIG. 1

# FIG. 2

0263716

# FIG. 3